# EUROPEAN PATENT APPLICATION

(11) **EP 4 336 489 A1**
(43) Date of publication of application: **13.03.2024**
(21) Application number: 23191223.9
(22) Date of filing: 13.08.2023
(51) Int. Cl.: G10K 15/02, A61M 21/02, G01H 3/12, H03G 3/32

(54) **SLEEP SOUND SYSTEM WITH ADAPTIVE ENVIRONMENT AUTOMATIC ADJUSTMENT**

(30) Priority: 08.09.2022 CN 202211097907
(71) Applicant: Zhongshan City Richsound Electronic Industrial Ltd, Zhongshan City Guangdong Province (CN)
(72) Inventor: Shen, Yang, Shenzhen City, Guangdong Province (CN)
(74) Representative: Jannig & Repkow Patentanwälte PartG mbB

(57) **Abstract**

The present disclosure discloses a sleep sound system with adaptive environment automatic adjustment, which comprises: a power supply module, which is connected with an external power supply; a main control module, which is connected with the power supply module; an environmental noise detection module, which is connected with the power supply module and the main control module and is configured to detect real-time environmental noise level; a storage module, which is connected with the power supply module and the main control module and is configured to store different audio data; and a power amplifier module, which is connected with the power supply module, the main control module and a loudspeaker. Through the above structure, the playing volume of sound can automatically follow the environmental noise level, and people can fall asleep better in the imitation of nature.

## Description

### TECHNICAL FIELD

The present disclosure relates to the field of sound, in particular to a sleep sound system with adaptive environment and automatic adjustment.

### BACKGROUND

An ordinary sleep sound has the following problems.
1. Although the existing sleep sound can simulate sounds from nature (i.e., wind, rain, thunder and lightning, river, bird-singing, white noise, etc.), and can set the start-stop time by a timer switch, this method cannot achieve the ideal effect for some insomniacs, because these sounds have to match the environment. For example, the running water in nature must be in the natural environment, and can only achieve good effects when there is no other noise interference around. If the sound of running water is listened to together with other noises in the city (i.e., noise from automobiles, air conditioners and other ambient noises in big cities), there is no such effect as that heard in the wild. However, most people work in cities and cannot sleep in the suburbs every day.
2. Generally, when people listen to music, they will adjust the volume according to the surrounding environment. For example, they will turn down the volume in a quiet environment, while they will turn up the volume in noisy environment. Hence, the volume level needs to be a suitable one with reference to the real-time surrounding sound. The existing sleep sound does not offer automatic volume adjustment feature at any time when people sleep.
3. A research team led by Professor Zhang Zhi from University of Science and Technology of China, Professor Tao Wenjuan from Anhui Medical University and Professor Yuanyuan Liu from the National Institutes of Health (NIH) explored a neural mechanism behind sound analgesia with experimental mice, and found that the effect of sound analgesia depends on the signal-to-noise ratio of relative environmental noise.

The researchers played classical music with harmonious melody, discordant note combinations and white noise, respectively, which turned out not so unexpectedly that "casting pearls before swine": the mice did not show different responses to several different types of sounds. However, when the sound intensity of any of these three sounds was only 5 decibels higher than the background noise, it seemed to have the effect of relieving pain. From the behavior of mice, their responses to avoiding nociceptive feelings decreased, and the negative emotions related to pain were also suppressed. Moreover, repeating these low-intensity sounds allowed the analgesic effect to last for several days and inhibit pain and inflammatory hypersensitivity. If the sound is more "strong", such as 10 decibels, 15 decibels and 20 decibels higher than the background noise, the sound will not help the mice effectively suppress the pain. It can be seen that when the sound is 5 decibels higher than the background noise, the sound creates a positive effect on enhancing sleeping and emotional relieve, but the existing sleep sound does not pay attention to this aspect.

Therefore, a sleep sound system with adaptive environment automatic adjustment can solve the above problems.

### SUMMARY

The present disclosure aims at solving at least one of the technical problems existing in the prior art. Therefore, the present disclosure provides a sleep sound system with adaptive environment and automatic adjustment.

The technical scheme adopted by an embodiment of the present disclosure to solve the technical problems is a sleep sound system with adaptive environment automatic adjustment, comprising:
a power supply module, which is connected with an external power supply;
a main control module, which is connected with the power supply module;
an environmental noise detection module, which is connected with the power supply module and the main control module and is configured to detect real-time environmental noise level;
a storage module, which is connected with the power supply module and the main control module and is configured to store different audio data; and
a power amplifier module, which is connected with the power supply module, the main control module and a loudspeaker.

Further, the environmental noise detection module comprises a microphone M1, capacitors C36-38 and a resistor R43, one end of the resistor R43 is connected with the main control module, the other end of the resistor R43 is connected with one end of the capacitor C37, one end of the capacitor C38 and the VDD end of the microphone M1, the other end of the capacitor C37 is connected with the other end of the capacitor C38 and the GND end of the microphone M1, respectively, and the OUT end of the microphone M1 is connected with the main control module through the capacitor C38.

Further, the microphone M1 is provided as a digital microphone.

Further, the sleep sound system with adaptive environment automatic adjustment further comprises an echo cancellation module, which is connected with the main control module, the power amplifier module and the loudspeaker, respectively.

Further, the echo cancellation module comprises resistors R5-6 and R29-30, one end of the resistor R5 is connected with the main control module and one end of the resistor R29, respectively, the other end of the resistor R5 is connected with the power amplifier module and the loudspeaker, respectively, one end of the resistor R6 is connected with the main control module and one end of the resistor R30, respectively, the other end of the resistor R6 is connected with the power amplifier module and the loudspeaker, respectively, and the other end of the resistor R29 and the other end of the resistor R30 are grounded.

The present disclosure has the following beneficial effects. The present disclosure discloses a sleep sound system with adaptive environment automatic adjustment, which comprises: a power supply module, which is connected with an external power supply; a main control module, which is connected with the power supply module; an environmental noise detection module, which is connected with the power supply module and the main control module and is configured to detect real-time environmental noise level; a storage module, which is connected with the power supply module and the main control module and is configured to store different audio data; and a power amplifier module, which is connected with the power supply module, the main control module and a loudspeaker. Through the above structure, the playing volume of sound can automatically follow the environmental noise level, and people can fall asleep better in the imitation of nature.

### BRIEF DESCRIPTION OF THE DRAWINGS

The above and/or additional aspects and advantages of the present disclosure will be apparent and easily understood from the description of the embodiments taken in conjunction with the following drawings, in which:
FIG. 1 is a schematic block diagram of a sleep sound system with adaptive environment and automatic adjustment.
FIG. 2 is a partial circuit diagram of a sleep sound system with adaptive environment automatic adjustment.
FIG. 3 is a circuit diagram of a main control module.
FIG. 4 is a circuit diagram of a memory module.
FIG. 5 is a circuit diagram of an environmental noise detection module.
FIG. 6 is a circuit diagram of a power amplifier module.
FIG. 7 is a circuit diagram of an echo cancellation module.
FIG. 8 is a circuit diagram of a power supply module.

### DETAILED DESCRIPTION OF THE EMBODIMENTS

The specific embodiments of the present disclosure will be described in detail in this section. The preferred embodiments of the present disclosure are shown in the attached drawings, which serves to supplement the description in the written part of the specification with figures, so that people can intuitively and vividly understand each technical feature and the overall technical scheme of the present disclosure, but it should not be understood as limiting the scope of protection of the present disclosure.

In the description of the present disclosure, "a plurality of" means more than two, and "greater than, less than, more than, etc." are understood as excluding this number, and "above, below, within, etc." are understood as including this number. If the first and second descriptions are only used for the purpose of distinguishing technical features, the descriptions cannot be understood as indicating or implying relative importance, implicitly indicating the number of indicated technical features, or implicitly indicating the sequence of indicated technical features.

In the description of the present disclosure, it should be understood that for the orientational description, the orientational or positional relationships indicated by the terms such as "up", "down", "front", "back", "left" and "right" are based on the orientational or positional relationships shown in the drawings only for the convenience of describing the present disclosure and simplifying the description, rather than indicate or imply that the referred devices or elements must have a specific orientation, be constructed and operated in a specific orientation, and therefore should not be construed as limiting the present disclosure.

In the present disclosure, unless otherwise defined expressly, the terms such as "provide", "mount" and "connect" should be understood broadly, for example, it can be direct connection or indirect connection through an intermediate medium; fixed connection, detachable connection or integral connection; mechanical connection; or the internal communication of two elements or the interaction between two elements. Those skilled in the art can reasonably determine the specific meaning of the above terms in the present disclosure in combination with the specific content of the technical scheme.

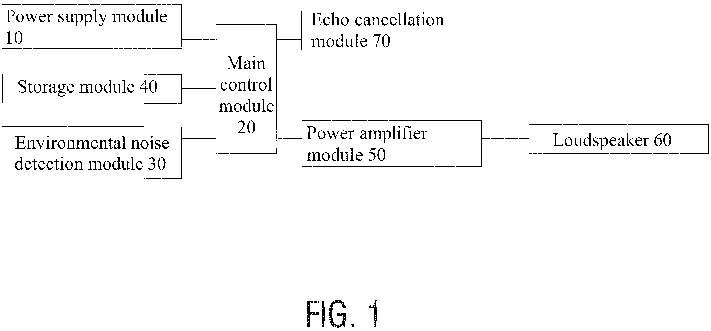

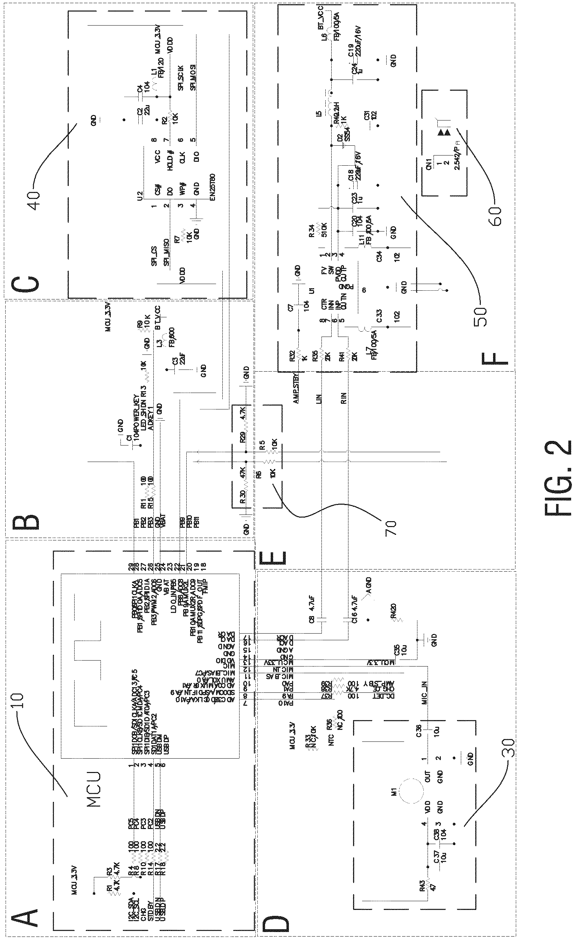

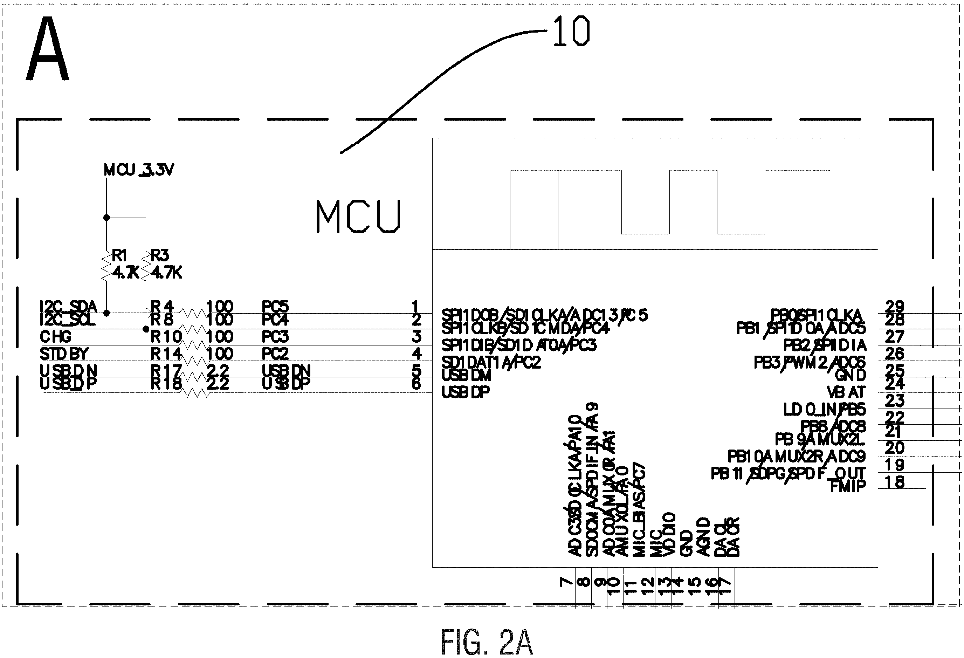

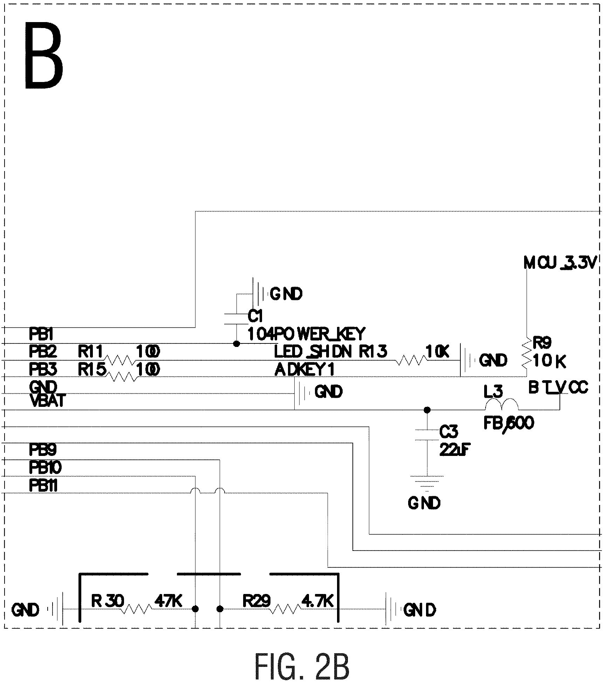

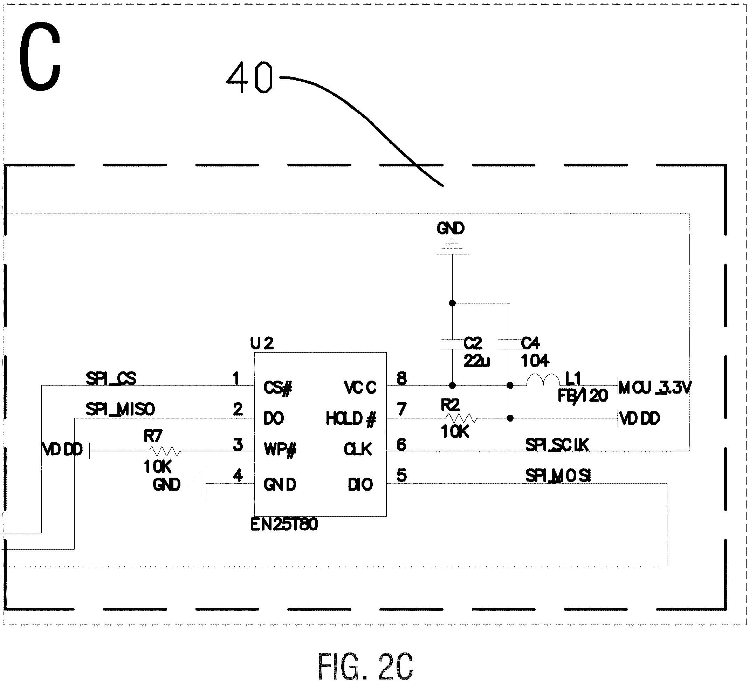

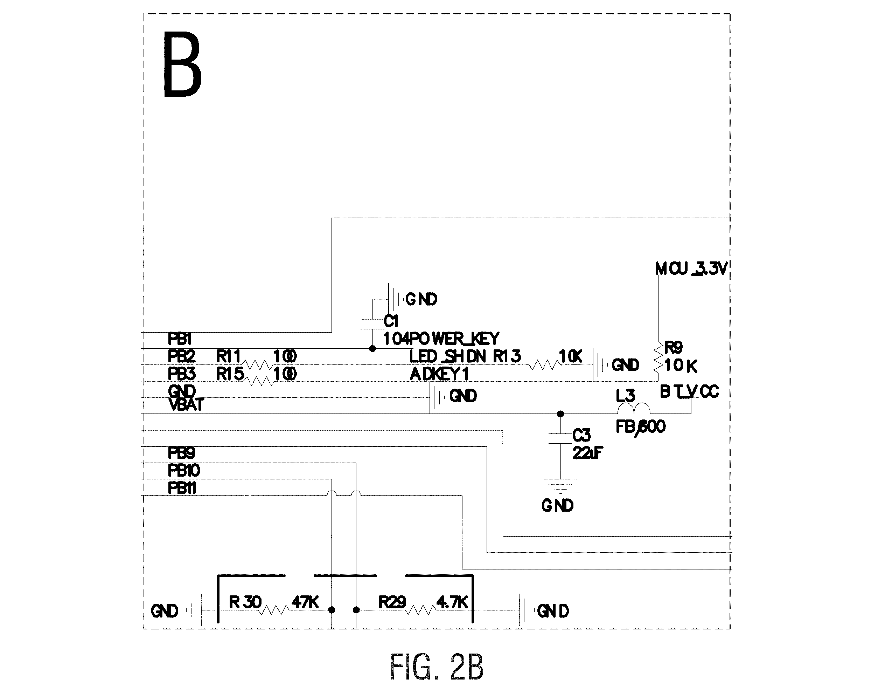

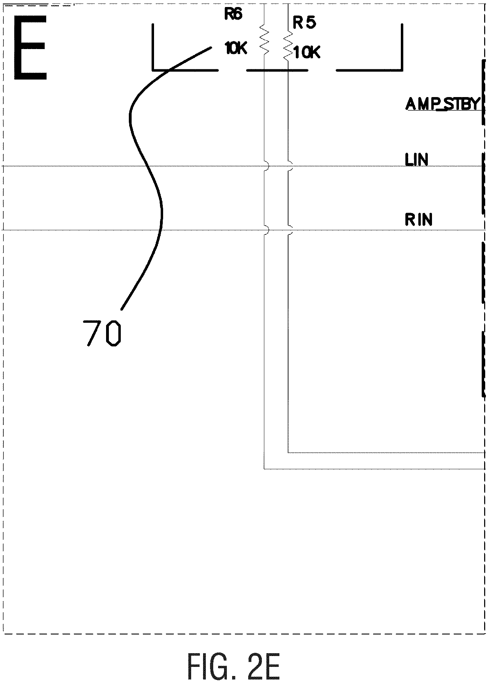

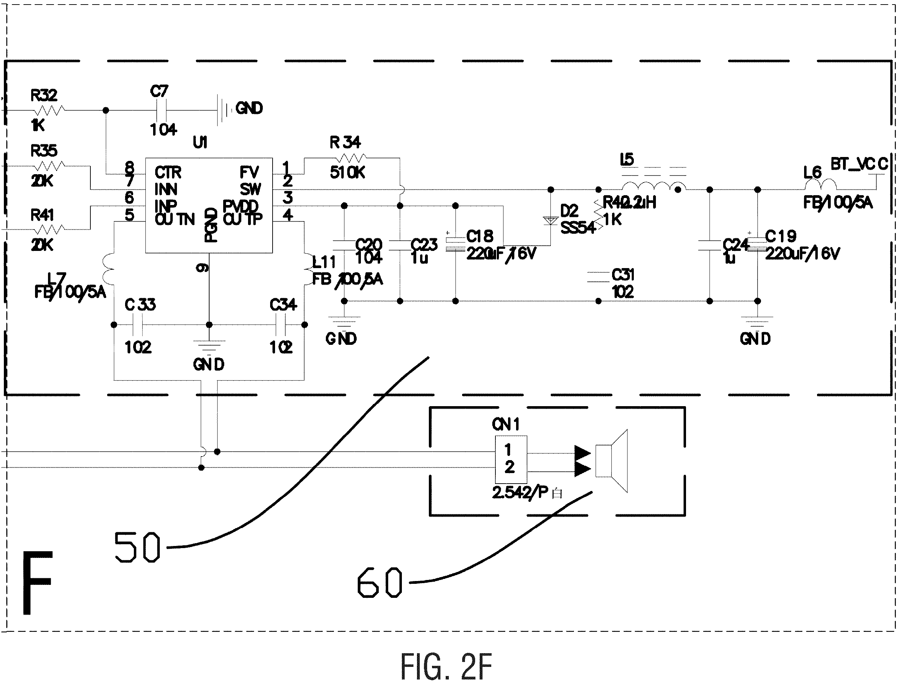

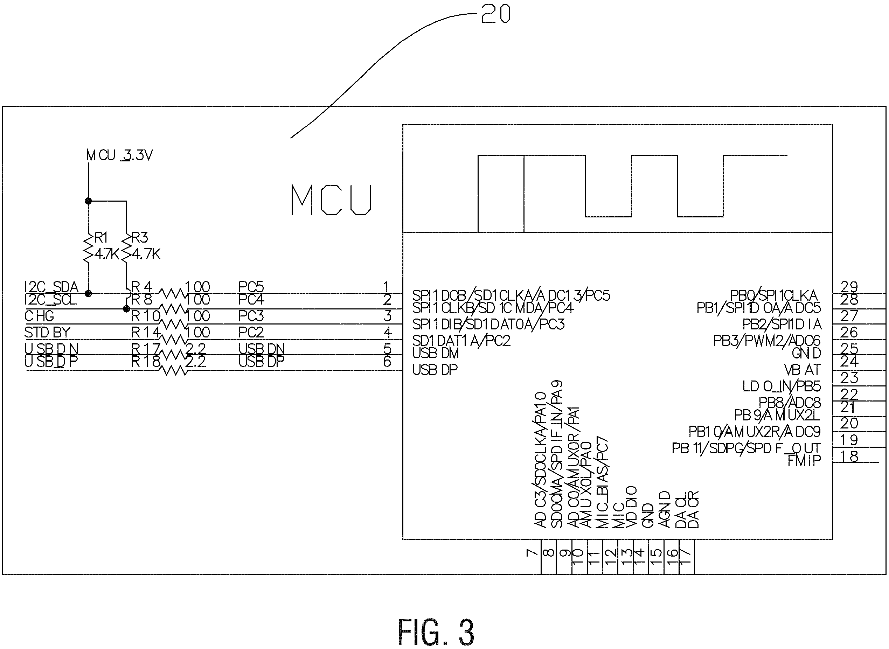

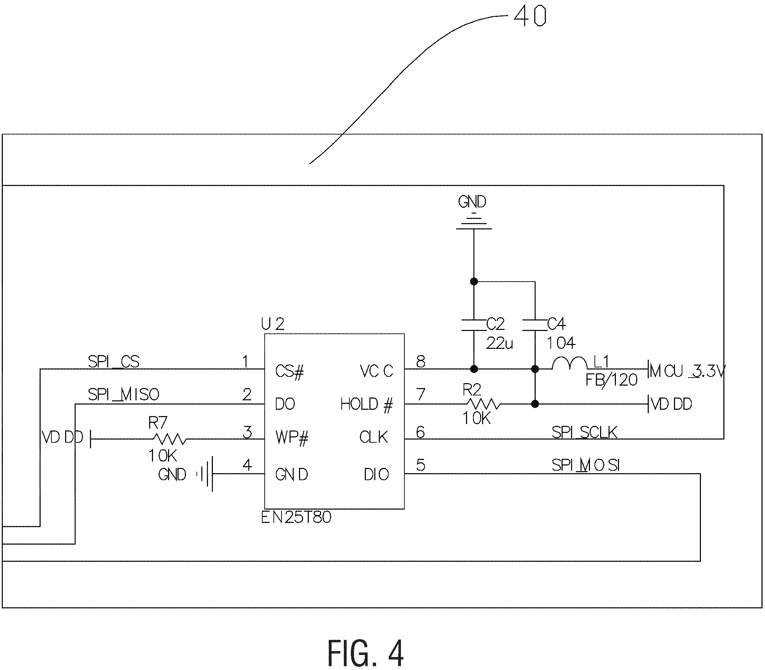

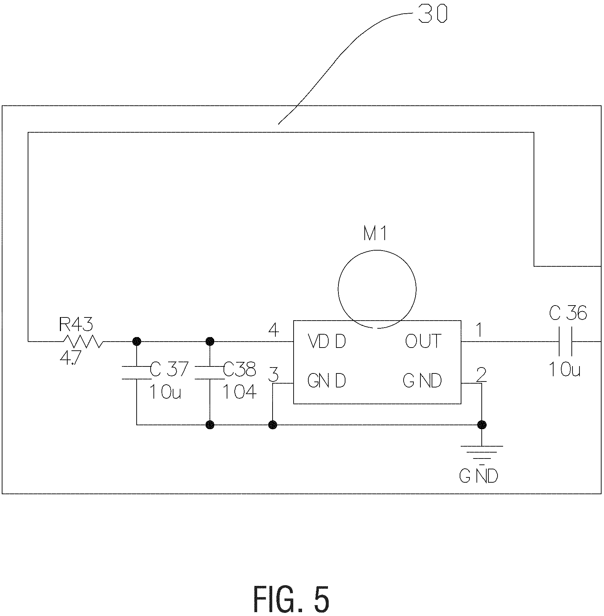

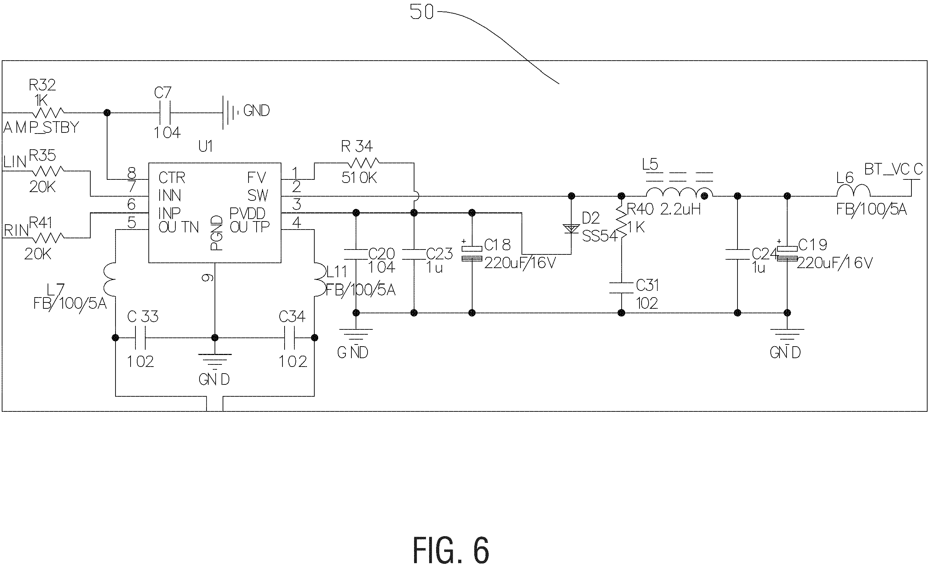

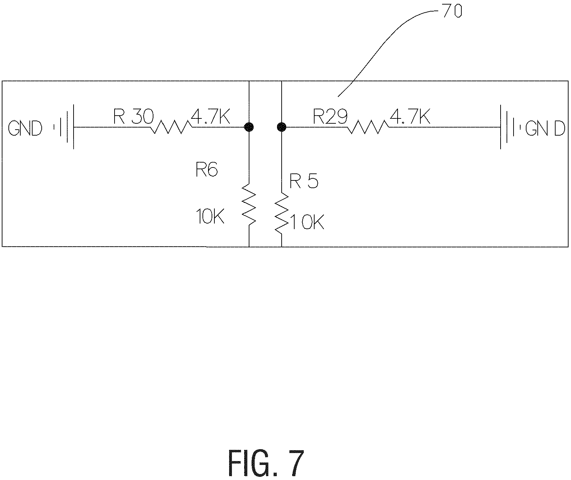

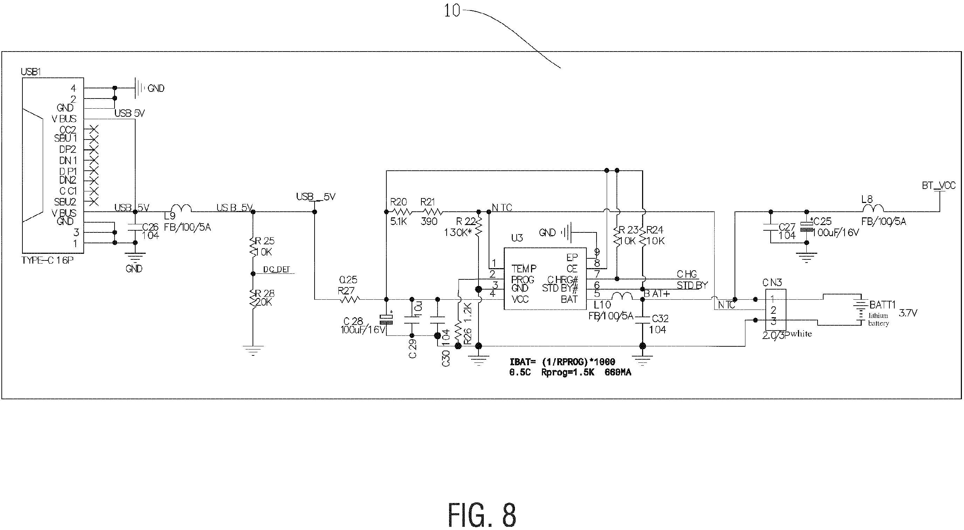

Referring to FIGS. 1 to 8, a sleep sound system with adaptive environment automatic adjustment comprises:
a power supply module 10, which is connected with an external power supply;
a main control module 20, which is connected with the power supply module 10;
an environmental noise detection module 30, which is connected with the power supply module 10 and the main control module 20 and is configured to detect real-time environmental noise level;
a storage module 40, which is connected with the power supply module 10 and the main control module 20 and is configured to store different audio data; and
a power amplifier module 50, which is connected with the power supply module 10, the main control module 20 and a loudspeaker 60.

In the present disclosure, the main control module 20 mainly comprises a Bluetooth chip with MCU function. The environmental noise detection module 30 mainly comprises a microphone M1 for detecting environmental noise. Specifically, the microphone M1 picks up the environmental noise signal and inputs the signal to the main control module 20 through the capacitor C36. After many experiments, it can be found that the volume is the most suitable when the volume is controlled at 5dB higher than the environmental sound, and people fall asleep the most easily when listening to music with this volume. Therefore, the main control module 20 takes this level as a reference, and then adjusts the played music level according to the environmental reference level +5dB and outputs the level to the power amplifier module 50 through the capacitors C8, C16, R35 and R41. The power amplifier module 50 drives the loudspeaker 60 to emit sound. When the environmental noise is reduced, the volume is also automatically reduced, thus achieving the purpose of automatic follow-up. Further, the storage module 40 mainly comprises a Flash chip U2, which is used to store audio data, including audio such as wind, rain, thunder and lightning, running water, and birdsong, so that users can fall asleep in the imitation of nature. In addition, if the sound emitted by the loudspeaker 60 is also received by the environmental noise detection module 30, the sound will interfere with the working mode therein, and the main control module 20 cannot distinguish whether the sound is environmental noise or music sound emitted by its own loudspeaker 60. In order to solve this problem, an echo cancellation module 70 consisted of circuits such as R5, R6, R29, and R30 is added. The sound emitted by the loudspeaker 60 is sampled by R1 and R2 and enters the main control module 20, and is compared with the level signal received by the environmental noise detection module 30. If the sound is the same as the level signal, it means that the sound is emitted by the loudspeaker 60, and the level signal is shielded to distinguish whether the sound is environmental noise or a music signal emitted by its own loudspeaker 60.

The environmental noise detection module 30 comprises a microphone M1, capacitors C36-38 and a resistor R43. One end of the resistor R43 is connected with the main control module 20, and the other end of the resistor R43 is connected with one end of the capacitor C37, one end of the capacitor C38 and the VDD end of the microphone M1. The other end of the capacitor C37 is connected with the other end of the capacitor C38 and the GND end of the microphone M1, respectively. The OUT end of the microphone M1 is connected with the main control module 20 through the capacitor C38.

The microphone M1 is provided as a digital microphone. Of course, the microphone M1 can also be a microphone for simulating signals. After receiving the signals, the microphone carries out analog-to-digital conversion, and then sends the signals to the main control module 20.

A sleep sound system with adaptive environment automatic adjustment further comprises an echo cancellation module 70, which is connected with the main control module 20, the power amplifier module 50 and the loudspeaker 60, respectively.

The echo cancellation module 70 comprises resistors R5-6 and R29-30. One end of the resistor R5 is connected with the main control module 20 and one end of the resistor R29, respectively, and the other end of the resistor R5 is connected with the power amplifier module 50 and the loudspeaker 60, respectively. One end of the resistor R6 is connected with the main control module 20 and one end of the resistor R30, respectively, and the other end of the resistor R6 is connected with the power amplifier module 50 and the loudspeaker 60, respectively. The other end of the resistor R29 and the other end of the resistor R30 are grounded.

Of course, the present disclosure is not limited to the above embodiments. Those skilled in the art can also make equivalent modifications or substitutions without departing from the spirit of the present disclosure, and these equivalent modifications and substitutions are all included in the scope defined by the claims of the present disclosure.

## Claims

1. A sleep sound system with adaptive environment automatic adjustment, comprising:
a power supply module (10), which is connected with an external power supply;
a main control module (20), which is connected with the power supply module (10);
an environmental noise detection module (30), which is connected with the power supply module (10) and the main control module (20) and is configured to detect real-time environmental noise level;
a storage module (40), which is connected with the power supply module (10) and the main control module (20) and is configured to store different audio data; and
a power amplifier module (50), which is connected with the power supply module (10), the main control module (20) and a loudspeaker (60).

2. The sleep sound system with adaptive environment automatic adjustment according to claim 1, wherein the environmental noise detection module (30) comprises a microphone M1, capacitors C36-38 and a resistor R43, one end of the resistor R43 is connected with the main control module (20), the other end of the resistor R43 is connected with one end of the capacitor C37, one end of the capacitor C38 and the VDD end of the microphone M1, the other end of the capacitor C37 is connected with the other end of the capacitor C38 and the GND end of the microphone M1, respectively, and the OUT end of the microphone M1 is connected with the main control module (20) through the capacitor C38.

3. The sleep sound system with adaptive environment automatic adjustment according to claim 2, wherein the microphone M1 is provided as a digital microphone.

4. The sleep sound system with adaptive environment automatic adjustment according to claim 1, further comprising an echo cancellation module (70), which is connected with the main control module (20), the power amplifier module (50) and the loudspeaker (60), respectively.

5. The sleep sound system with adaptive environment automatic adjustment according to claim 4, wherein the echo cancellation module (70) comprises resistors R5-6 and R29-30, one end of the resistor R5 is connected with the main control module (20) and one end of the resistor R29, respectively, the other end of the resistor R5 is connected with the power amplifier module (50) and the loudspeaker (60), respectively, one end of the resistor R6 is connected with the main control module (20) and one end of the resistor R30, respectively, the other end of the resistor R6 is connected with the power amplifier module (50) and the loudspeaker (60), respectively, and the other end of the resistor R29 and the other end of the resistor R30 are grounded.
